(19) Europäisches Patentamt
European Patent Office
Office européen des brevets

(11) EP 1 141 391 B1

(12) FASCICULE DE BREVET EUROPEEN

(45) Date de publication et mention de la délivrance du brevet:
**02.03.2005 Bulletin 2005/09**

(21) Numéro de dépôt: **99958329.7**

(22) Date de dépôt: **15.12.1999**

(51) Int Cl.⁷: **C12Q 1/68**, G01N 33/543

(86) Numéro de dépôt international:
**PCT/FR1999/003141**

(87) Numéro de publication internationale:
**WO 2000/036145 (22.06.2000 Gazette 2000/25)**

# (54) PROCEDE DE FABRICATION D'UNE BIOPUCE ET BIOPUCE

VERFAHREN ZUR HERSTELLUNG EINES BIOCHIP SOWIE BIOCHIP

METHOD FOR MAKING A BIOCHIP AND BIOCHIP

(84) Etats contractants désignés:
**AT BE CH CY DE DK ES FI FR GB GR IE IT LI LU MC NL PT SE**

(30) Priorité: **16.12.1998 FR 9815883**

(43) Date de publication de la demande:
**10.10.2001 Bulletin 2001/41**

(73) Titulaire: **COMMISSARIAT A L'ENERGIE ATOMIQUE**
**75752 Paris Cédex 15 (FR)**

(72) Inventeurs:
- **ROSILIO, Charles**
**F-91190 Gif-sur-Yvette (FR)**
- **CAILLAT, Patrice**
**F-38130 Echirolles (FR)**

(74) Mandataire: **Audier, Philippe André et al**
**Brevatome,**
**3, rue du Docteur Lancereaux**
**75008 Paris (FR)**

(56) Documents cités:
**EP-A- 0 038 244**
**EP-A- 0 229 993**
**EP-A- 0 588 721**
**EP-A- 0 659 794**
**US-A- 5 653 939**
**US-A- 5 810 989**
**US-A- 5 837 859**

- **LIVACHE T ET AL.: "Preparation of a DNA matrix via an electrochemically directed copolymerization of pyrrole and oligonucleotides bearing a pyrrole group" NUCLEIC ACIDS RESEARCH, vol. 22, no. 15, 1994, pages 2915-2921, XP002114812**
- **LIVACHE T ET AL.: "Electroconducting polymers for the construction of DNA or peptide arrays on silicon chips" BIOSENSORS & BIOELECTRONICS, vol. 13, 1998, pages 629-634, XP000869823**
- **LIVACHE T ET AL.: "Polypyrrole DNA chip on a silicon device: Example of hepatitis C virus typing" ANALYTICAL BIOCHEMISTRY, vol. 255, 1998, pages 188-194, XP002114813**
- **SIMON R A ET AL.: "Synthesis and characterization of a new surface derivatizing reagent to promote the adhesion of polypyrrole films to n-type silicon photoanodes: N-(3-(Trimethoxysilyl)propyl)pyrrole" JOURNAL OF THE AMERICAN CHEMICAL SOCIETY, vol. 104, 1982, pages 2031-2034, XP002114814 cited in the application**
- **GUISEPPI-ELIE A AND WILSON A M: "Specific immobilization of electropolymerized polypyrrole thin films onto interdigitated microsensor electrode arrays" LANGMUIR, vol. 11, 1995, pages 1768-1776, XP002114815**
- **NISHIZAWA M ET AL.: "Electrochemical preparation of ultrathin polypyrrole film at microarray electrodes" JOURNAL OF PHYSICAL CHEMISTRY, vol. 95, 1991, pages 9042-9044, XP002114816**
- **BIDAN G ET AL.: "Conducting polymers as a link between biomolecules and microelectronics" SYNTHETIC METALS, vol. 102, 1999, pages 1363-1365, XP002114817**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

### Domaine technique de l'invention

La présente invention se rapporte à un procédé de fabrication d'une biopuce et à une biopuce, ladite biopuce étant constituée notamment de sondes biologiques greffées sur un polymère conducteur.

Les dispositifs d'analyse biologique, par exemple de type puce à ADN, constituent des outils performants pour l'analyse en parallèle d'un grand nombre de gênes ou de séquences d'ADN ou d'ARN. Leur principe de fonctionnement repose sur la propriété d'hybridation ou d'appariement de deux brins de séquences complémentaires afin de reconstituer la double hélice d'ADN. Pour ce faire, des sondes d'oligonucléotides de séquence connue, immobilisées sur un substrat support, sont mises en présence de cibles extraites d'un échantillon biologique à analyser, et marquées à l'aide de marqueurs fluorescents.

L'hybridation est ensuite repérée et la séquence détectée par analyse de la surface de la puce par un marqueur approprié par exemple permettant de détecter la séquence par fluorescence.

Des technologies très différentes ont été utilisées pour la réalisation de ces matrices de sondes. Diverses techniques d'immobilisation ou de greffage des sondes sur des substrats différents ont fait l'objet d'études et de développements industriels importants.

### Etat de l'art antérieure

Il existe principalement trois méthodes d'adressage de sondes chimiques qui constituent des approches différentes de réalisation et d'utilisation de sondes pour différents domaines d'application. Il s'agit de l'adressage photochimique, de l'adressage mécanique, par exemple par micropipetage à l'aide d'un robot disperseur, et de l'adressage électrochimique.

Par exemple, l'adressage électrochimique peut être utilisé pour les sondes d'oligonucléotides. Pour ce faire, des matrices d'électrodes adressées individuellement sont réalisées sur un substrat de verre.

Le principe d'immobilisation des sondes biologiques repose sur un dépôt par électropolymérisation d'un copolymère de pyrrole et de pyrrole substitué par un oligonucléotide (Py-ODN), portant un oligonucléotide greffé sur un noyau de pyrrole soit directement, soit indirectement par l'intermédiaire d'un espaceur.

Dans le but de développer des systèmes d'analyse biologique massivement parallèles, à grande capacité ou densité de sites actifs, il est nécessaire de pouvoir adresser individuellement un nombre important de sondes.

Les procédés utilisant un adressage électrochimique nécessitent à la fois une matrice importante d'électrodes et de connexions et un multiplexeur pour indexer électriquement chacun des sites de la puce. De plus, dans ces procédés, il faut réaliser l'électropolymérisation par trempage de la puce entière successivement dans des solutions de chacun des Py-ODN contenus dans la cellule. Ces procédés sont donc limités à des puces de faible densité, c'est-à-dire d'environ une centaine de sondes, pour des applications limitées et spécifiques.

D'autres procédés ont encore été décrits dans l'art antérieur remplaçant avantageusement l'adressage électrique individuel par un adressage mécanique. Il reste cependant un inconvénient, celui de réaliser des électropolymérisations dans des microcuvettes, avec un volume de solution de l'ordre du nanolitre, pour lequel il est nécessaire de retarder l'évaporation après micropipetage de l'ensemble des sondes sur la plaquette afin que l'électropolymérisation puisse se faire.

Le document US-A-5 837 859 décrit une biopuce comprenant un ensemble de microélectrodes sur lesquelles sont électropolymérisés du pyrrole et du pyrrole fonctionnalisé. Le document Nucleic Acids Research, vol. 22(15), 1994, pp. 2915-2921 décrit la préparation d'une matrice d'ADN par une copolymérisation électrochimique de pyrrole et d'oligonucléotides portant un groupe pyrrole sur une multitude d'électrodes formées par des technologies de microélectronique. Les documents Biosensor & Bioelectronics, vol. 13, 1998, pp. 629-634 and Analytical Biochemistry, vol. 255, 1998, pp. 188-194 décrivent des polymères électroconducteurs pour la construction de matrices d'ADN ou de peptides sur une puce à base de silicium. Le support comprend une matrice de microélectrodes formées sur un substrat de silicium par des technologies de microélectronique. Le document Langmuir, vol. 11, 1995, pp. 1768-1776 décrit un procédé pour identifier des structures moléculaires dans un échantillon. En particulier, il décrit une modification chimique permettant d'augmenter l'adhésion d'un film de polypyrrole formé sur des matrices d'électrodes interdigitées d'or sur du verre borosilicaté et/ou du silicium oxydé.

### Exposé de l'invention

La présente invention a précisément pour but de résoudre les problèmes précités en fournissant un procédé de fabrication d'une biopuce constituée notamment de sondes biologiques greffées sur un polymère conducteur, ledit procédé présentant notamment l'avantage de ne nécessiter l'utilisation que d'une seule solution d'un mélange en

proportion adéquate de pyrrole et de pyrrole substitué (Py et Py-R-F ou F et une fonction chimique réactive et R est un groupement espaceur aliphatique ou aromatique) pour une seule électrodéposition collective sur l'ensemble des microcuvettes.

Le procédé de l'invention se caractérise en ce qu'il comprend les étapes suivantes :

a) structuration d'un substrat de manière à obtenir sur ce substrat des microcuvettes comprenant dans leur fond une couche d'un matériau capable d'initier et de promouvoir l'adhésion sur celle-ci d'un film d'un copolymère de pyrrole et de pyrrole fonctionnalisé par électropolymérisation.

b) électropolymérisation collective, de manière à former un film électropolymérisé d' un copolymère de pyrrole et de pyrrole fonctionnalisé sur le fond desdites microcuvettes, sur la couche dudit matériau, à partir d'une solution de pyrrole et de pyrrole fonctionnalisé, en présence de réactifs chimiques appropriés pour ladite électropolymérisation,

c) fixation directe, ou indirecte, d'une sonde biologique sur le pyrrole fonctionnalisé, par injection d'une solution de la sonde biologique, au choix dans une ou plusieurs microcuvette(s) en présence de réactifs chimiques nécessaires à la fixation directe, ou indirecte, de cette sonde biologique sur le pyrrole fonctionnalisé.

Selon l'invention, la couche du matériau capable d'initier et de promouvoir l'adhésion du film de copolymère de pyrrole et de pyrrole fonctionnalisé par électropolymérisation sur celle-ci peut être une couche métallique, l'étape a) précitée pouvant alors comprendre une étape de dépôt de ladite couche métallique sur le substrat, et une étape de dépôt d'une couche de résine ou de polymère sur la couche métallique et de développement ou de gravure de ladite couche de manière à former les microcuvettes dont le fond est constitué au moins en partie de la couche métallique.

Selon l'invention, la couche métallique peut être par exemple une couche d'or, une couche de cuivre ou d'argent ou d'aluminium.

Selon l'invention, le substrat peut être par exemple une plaquette de silicium, une plaquette de verre ou un support plastique flexible si nécessaire.

Selon un autre mode de réalisation de la présente invention, l'étape a) peut comprendre en outre une étape de traitement de la couche d'or au fond des microcuvettes en présence d'un pyrrole fonctionnalisé par exemple avec un groupement thiol de manière à former une monocouche de pyrrole sur ladite couche métallique, par exemple sur ladite couche d'or, au fond desdites microcuvettes. Cette monocouche est capable d'initier et de promouvoir l'adhésion d'un film de polypyrrole par électropolymérisation comme l'ont montré R. Simon et coll. (J. Am. Chem. Soc., 1982, 104, 2031). Il s'agit d'une monocouche auto-assemblée (SAM) d'un pyrrole fonctionnalisé pour son accrochage sur le fond des microcuvettes.

Selon l'invention, le pyrrole fonctionnalisé peut être un pyrrole qui présente un groupement chimique permettant sa fixation par liaison covalente avec la couche métallique, et/ou avec la sonde biologique. Dans le cas de sa fixation à la couche métallique, par exemple à la couche d'or, un pyrrole fonctionnalisé avec un groupement thiol ou disulfure peut également être utilisé.

Par exemple, le pyrrole fonctionnalisé avec un groupement thiol peut avoir la formule chimique suivante :

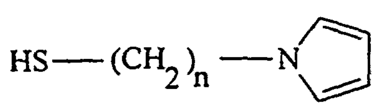

dans laquelle n peut avoir une valeur allant de 1 à 10, par exemple n peut être égal à 6.

Pour une électrode métallique en aluminium, on peut choisir un pyrrole fonctionnalisé avec un groupe -COOH.

Selon un autre mode de réalisation de la présente invention, le substrat peut être une plaquette de silicium et la couche capable d'initier et de promouvoir l'adhésion sur celle-ci d'un film de polypyrrole par électropolymérisation, peut être une couche de silane présentant un alignement de sites pyrroles. L'étape a) du procédé de la présente invention peut alors comprendre une étape de dépôt d'une couche de résine sur la plaquette de silicium, ladite plaquette de silicium étant recouverte d'un film de $SiO_2$, et de gravure de ladite couche de résine de manière à former les microcuvettes dont le fond est constitué au moins en partie du film de $SiO_2$ ; et une étape de traitement des microcuvettes au moyen d'un agent de silanisation fonctionnalisé avec un pyrrole de manière à fixer, sur le film de $SiO_2$, dans le fond des microcuvettes la couche de silane présentant un alignement de sites pyrroles.

Selon l'invention, l'agent de silanisation peut être choisi dans un groupe comprenant le N-(3-(triméthoxy silyl) propyl) pyrrole, ou tout autre pyrrole fonctionnalisé avec un groupement $-SiCl_3$ ou $-Si(OMe)_3$. Le film de $SiO_2$ peut être un film naturel de $SiO_2$ présent sur les plaquettes de silicium.

Selon l'invention, quel que soit le mode de réalisation, la résine peut être une résine photosensible, dont le masquage, l'insolation et le développement permettent de former les microcuvettes.

Selon l'invention, l'électropolymérisation collective de l'étape b) du procédé peut être par exemple réalisée par trempage du substrat structuré obtenu à l'étape a) précitée dans un bain électrolytique comprenant une solution de pyrrole, de pyrrole fonctionnalisé, et de réactifs chimiques appropriés pour l'électropolymérisation, en présence d'une contre-électrode à l'électrode de travail qui trempe dans le bain électrolytique et est indépendante du substrat structuré.

Selon l'invention, dans cette étape b), le pyrrole fonctionnalisé peut être un pyrrole comportant un groupement choisi dans un ensemble comprenant un groupement $NH_2$, un groupement thiol, un groupement ester succinimide, un groupement triméthoxy silyl, un groupement carboxylique, aldéhyde et isothiocyanate.

Selon l'invention, le pyrrole fonctionnalisé par l'électropolymérisation peut par exemple être choisi parmi un des composés suivants :

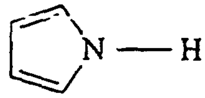

PYRROLE

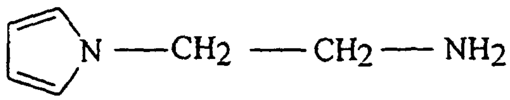

N-ETHYLAMINE PYRROLE

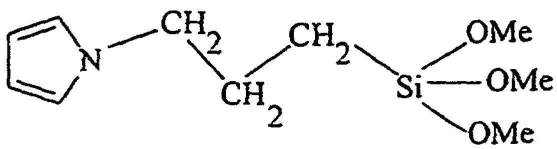

N(3-(TRIMETHOXY SILYL)PROPYL)PYRROLE

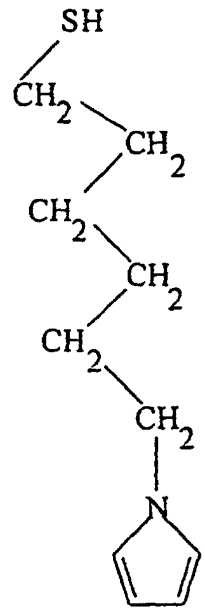

PYRROLE fonctionnalisé avec un thiol

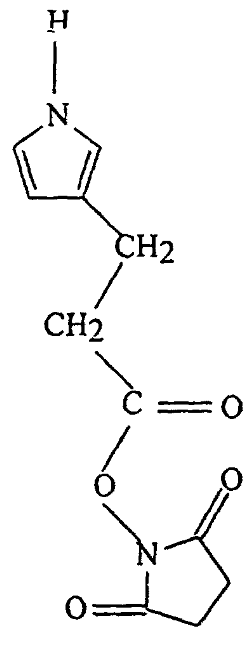

PYRROLE fonctionnalisé en 3' par un ester succinimydyl.

Selon l'invention, le bain électrolytique peut être un mélange de pyrrole et de pyrrole fonctionnalisé en proportions adéquates pour former un film présentant un nombre désiré d'unités de pyrrole fonctionnalisé. Ainsi, le procédé de l'invention permet de choisir le nombre de sondes biologiques par microcuvette, car selon ce procédé, les sondes biologiques sont fixées, soit directement, soit indirectement, sur ces pyrroles fonctionnalisés.

L'étape c) suivante du procédé de l'invention consiste en une fixation directe ou indirecte d'une sonde biologique sur le pyrrole fonctionnalisé.

Selon l'invention, lorsque la fixation de la sonde biologique est indirecte, l'étape c) du procédé de l'invention peut comprendre en outre, avant la fixation de la sonde biologique, une fixation collective d'un agent de réticulation sur le pyrrole fonctionnalisé, en présence de réactifs chimiques appropriés, ledit agent de réticulation comportant une première fonction permettant sa fixation sur le pyrrole fonctionnalisé, et une deuxième fonction permettant la fixation de la sonde biologique sur ledit agent de réticulation.
Selon l'invention, l'agent de réticulation peut par exemple être un agent de réticulation bifonctionnel.
L'agent de réticulation peut par exemple présenter une fonction ester de la N-hydroxysuccinimide et une fonction maléimide.
Selon l'invention, l'agent de réticulation peut par exemple être choisi parmi un des composés suivants :

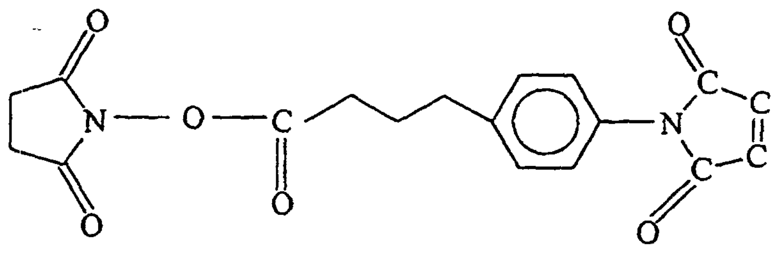

ester de N-hydroxysuccinimide fonction maléique

SMPB

succinimidyl 4-(p-maléimidophényl)butyrate

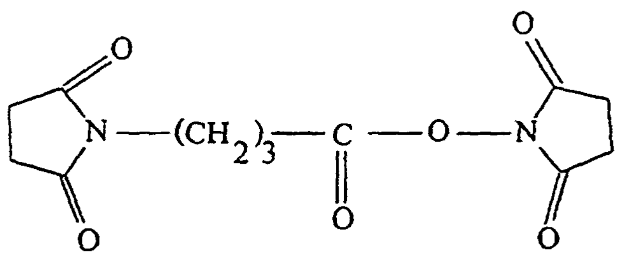

GMBS

N-maléimidobutyryloxy succinimide ester,

un dialdéhyde du type

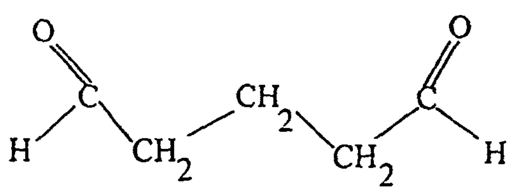

GLUTARALDEHYDE,

un diisothiocyanate du type

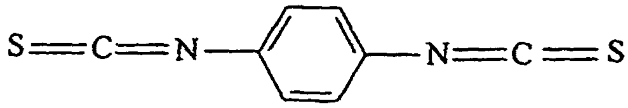

1,4-PHENYLENE DIISOTHIOCYANATE,

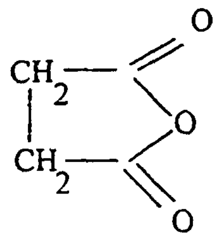

ANHYDRIDE SUCCINIQUE ou acide succinique

ou un dérivé de ces composés.

Tous les agents de réticulation bi-fonctionnels précités sont bien adaptés pour les polypyrroles fonctionnalisés avec le groupement $-CH_2-CH_2-NH_2$ en position 1 sur l'azote. Mais une électropolymérisation avec un pyrrole fonctionnalisé avec d'autres groupements sont aussi possibles. Par exemple Py-$CH_2$-$CH_2$-$NH_2$, Py-SH, Py-succinimidyl ester (en 3), Py-hydrazine avec une substitution en 1 sur l'azote ou en 3 sur le cycle pyrrole, permettant d'immobiliser les oligonucléotides, soit directement, soit par l'intermédiaire d'un agent de réticulation, par exemple bi-fonctionnel.

Les agents de réticulation suivants peuvent donc être utilisés dans le procédé de la présente invention :

a)un dialdéhyde du type glutaraldéhyde, qui peut réagir sur les fonctions $NH_2$ du film de polypyrrole (étape collective) puis sur la fonction $NH_2$ d'un oligonucléotide terminé par exemple par un phosphate portant un groupement aminé, par une étape individuelle dans les microcuvettes ;
b)un diisothiocyanate qui peut également réagir sur la fonction amine du polypyrrole
fonctionnalisé par une extrémité (étape collective) puis sur une fonction amine d'un oligonucléotide terminé par un phosphate avec un groupement espaceur fonctionnalisé avec $NH_2$ ;
c)un anhydride succinique qui par ouverture met en jeu deux fonctions acides capables de réagir sur les $NH_2$ du polypyrrole et d'autre part sur les $NH_2$ d'un oligonucléotide fonctionnalisé avec $NH_2$.

Selon l'invention, la sonde biologique qui va être à l'origine de la spécificité de la biopuce fabriquée, peut être choisie par exemple parmi un oligonucléotide, un ADN, un ARN, un peptide, un glucide, un lipide, une protéine, un anticorps, un antigène.

Selon l'invention la sonde biologique est de préférence fonctionnalisée pour pouvoir être fixée soit directement, soit indirectement sur le pyrrole fonctionnalisé. Cette fonctionnalisation a pour but de fixer sur la sonde biologique un groupement chimique capable de former une liaison covalente entre la sonde biologique et le pyrrole fonctionnalisé.

Elle peut être par exemple fonctionnalisée avec un groupement thiol, avec un groupement $NH_2$, aldéhyde, un groupement -COOH ou encore un groupement phsopahte acide.

Par exemple lorsque la sonde biologique est un oligonucléotide, elle peut être fonctionnalisée avec un groupement thiol (SH). Les oligonucléotides fonctionnalisés avec S-H peuvent être préparés selon une procédure connue, par exemple en fin d'une synthèse automatisée d'oligonucléotides.

Dans un cas où il est plus facile de disposer d'oligonucléotides fonctionnalisés avec $NH_2$, il est possible par exemple de synthétiser un pyrrole fonctionnalisé avec un S-H pour la copolymérisation, d'utiliser par exemple SMPB avec ses deux fonctions spécifiques et d'immobiliser les oligonucléotides fonctionnalisés avec $NH_2$ par liaison covalente avec la fonction succinamide de cet agent de réticulation.

Dans le cas d'oligonucléotides terminés en 3' par un nucléotide N-méthyl uridine, une réaction d'oxydation sur cette fonction permet d'obtenir un oligonucléotide fonctionnalisé avec une fonction aldéhyde, capable de réagir directement, c'est-à-dire par exemple sans l'agent bifonctionnel sur le polypyrrole fonctionnalisé avec $NH_2$.

Pour fonctionnaliser un oligonucléotide avec une fonction $NH_2$, l'une des méthodes utilisables selon le procédé de la présente invention peut consister à faire un couplage entre l'oligonucléotide et le N-trifluoroacétyl-6 amino hexyl-2 cyanoéthyl NN'-diisopropyl phosphoramidite disponible dans le commerce.

Par ailleurs, un oligonucléotide fonctionnalisé avec $NH_2$ peut par exemple être converti en oligonucléotide terminé par un thiol par une réaction avec le dithiobis (succinimidylpropionate).

Les oligonucléotides sondes fonctionnalisés peuvent par exemple être prélevés par micropipetage dans des micropuits et injectés dans les microcuvettes par exemple par l'intermédiaire d'un microrobot dispenseur ou par impression par jets. Ces appareils sont bien connus de l'homme du métier.

Le procédé de la présente invention permet avantageusement de choisir le nombre de sondes par site actif, c'est-à-dire par microcuvette en jouant sur la proportion de pyrrole fonctionnalisé par rapport au pyrrole.

La densité de sondes souhaitée peut être contrôlée par exemple par fixation d'oligonucléotides marquées en extrémité de chaînes par une biotine et en utilisant la reconnaissance par streptavidine-Cy3 par une analyse de surface de la puce par les méthodes classiques de détection par fluorescence.

Un autre avantage du procédé de l'invention réside dans le fait que les deux opérations collectives, électropolymérisation et éventuellement fixation de l'agent de réticulation, peuvent se faire par lots sur un grand nombre de plaquettes en parallèle.

Les plaquettes ayant subi les étapes a) et b) du procédé de l'invention sont aussi appelées "ébauches de biopuces". Elles sont prêtes à être soumises à l'étape de fixation directe ou indirecte d'une sonde biologique, par exemple d'un oligonucléotide conforme à la présente invention.

Ainsi, le procédé de l'invention permet par exemple de fabriquer une puce oligonucléotidique comprenant dans cet ordre :

- soit un substrat de silicium recouvert de silice, et d'une couche de silane fonctionnalisé avec des pyrroles,
- soit une couche d'or ou une couche silane présentant des sites pyrroles,
- soit une couche d'or avec ou sans une couche de promotion et d'adhérence de l'électropolymérisation (basée sur un pyrrole fonctionnalisé avec un thiol -SH),
- soit une couche d'aluminium avec un pyrrole fonctionnalisé avec un -COOH,
- et une couche de résine dans laquelle des microcuvettes ont été réalisées de telle sorte que le fond desdites microcuvettes est constitué au moins en partie de la couche d'or ou de la couche de silane présentant des sites pyrroles,
- et une couche d'un copolymère de pyrrole et de pyrrole fonctionnalisé, fixée sur la couche d'or ou la couche de silane présentant des sites pyrroles constituant le fond desdits microcuvettes, le pyrrole fonctionnalisé étant lié ou non à un agent de réticulation bifonctionnel,
- et un oligonucléotide fixé directement sur le pyrrole fonctionnalisé, ou indirectement sur le pyrrole fonctionnalisé par l'intermédiaire de l'agent de réticulation lié au pyrrole.

D'autres avantages et caractéristiques de la présente invention apparaîtront encore à la lecture de la description qui suit, donnée bien entendu à titre illustratif et non limitatif, en référence aux dessins en annexe.

Brève description des figures

- La figure 1 est un schéma d'une vue en coupe d'un substrat structuré selon un premier mode de réalisation des étapes a) et b) du procédé de la présente invention.
- La figure 2 est un schéma d'une vue en coupe d'un substrat structuré selon le mode de réalisation représenté sur la figure 1, et comprenant en outre un agent de réticulation pour une fixation indirecte d'une molécule biologique.
- La figure 3 est un schéma d'une vue en coupe d'un substrat structuré représenté sur la figure 2, illustrant la fixation indirecte d'un oligonucléotide sur l'agent de réticulation.
- La figure 4 est un schéma d'une vue en coupe d'un substrat structuré selon un second mode de réalisation du procédé de la présente invention.
- La figure 5 est un schéma d'une vue en coupe d'un substrat structuré selon un troisième mode de réalisation des étapes a) et b) du procédé de la présente invention.

## EXEMPLES

Exemple 1 : Fabrication d'une biopuce constituée notamment d'oligonucléotides greffés sur un polymère conducteur selon un premier mode de réalisation de la présente invention.

Selon ce premier mode de réalisation, relatif notamment à l'étape a) du procédé de l'invention, une couche d'or est déposée sur une plaquette de silicium de manière à former une électrode de travail pour l'électropolymérisation

d'un copolymère de pyrrole et de pyrrole fonctionnalisé. Cette couche d'or est déposée par une technique classique d'évaporation sous vide ou pulvérisation cathodique. Elle a une épaisseur d'environ 1000 à 5000 Å et constitue l'électrode de travail collective.

Une résine photosensible est déposée sur l'électrode en or et une étape de photolithographie permet de pratiquer des ouvertures dans la résine de manière à former des microcuvettes comportant l'électrode de travail dans leur fond, ces microcuvettes peuvent être adressées simultanément.

La résine utilisée est de préférence :

a)une résine photosensible de type positif (Novolaque + diézonaphtoquinone à développement en milieu alcalin) ;
b)une résine photosensible négative de type Polyimide (OLIN) à développement dans un solvant organique ;
c)soit un polymère gravé par gravure sèche ou humide.

Les microcuvettes formées ont une dimension de 100x100x30 μm.

La résine est déposée sur l'électrode en or par une technique classique de centrifugation à la tournette ("spinning"). Un substrat structuré selon l'étape a), du procédé de la présente invention est ainsi obtenu.

L'étape b) d'électropolymérisation collective est réalisée en utilisant une solution de pyrrole et de pyrrole fonctionnalisé.

Dans cet exemple, le pyrrole fonctionnalisé est le N-éthylaminepyrrole, et la solution utilisée pour l'électropolymérisation est une solution aqueuse/éthanol ou acétonitrile comprenant 0,1 mole de pyrrole, et un rapport molaire pyrrole fonctionnalisé/pyrrole de 5% à 0,5%en poids de pyrrole fonctionnalisé. Cette solution est appelée ci-après bain électrolytique.

L'obtention du monomère de pyrrole fonctionnalisé avec une fonction $NH_2$ est aisée et est décrite par exemple dans I. Jirkowsky, R. Baudy, Synthesis 1981, p. 481.

L'électropolymérisation est réalisée par trempage dans le bain électrolytique du substrat structuré obtenu précédemment, avec des réactifs appropriés pour l'électrochimie. Ces réactifs sont par exemple des sels électrolytiques ($Li^+ClO_4^-$, sels d'ammonium quaternaires, Li-toxylate, ou du polystyrène sulfonate de lithium, de potassium ou de sodium).

Les solvants pour l'électropolymérisation sont, par exemple, le $CA_3CN$, l'eau, l'éthanol et les mélanges eau-éthanol. Le pyrrole contenu dans le bain présente une concentration de l'ordre de $10^{-1}$ à $10^{-3}$ M/l.

Une contre-électrode en platine et une électrode de référence au calomel trempent dans le bain électrolytique et sont indépendantes de la plaquette de silicium, seule l'électrode de travail est intégrée à la structure de la plaquette.

Un film de copolymère de pyrrole et de pyrrole fonctionnalisé est ainsi formé et déposé uniquement sur le fond des microcuvettes par électrodéposition.

La figure 1 est un schéma d'une vue en coupe du substrat obtenu selon ce premier mode de réalisation du procédé de la présente invention. Sur cette figure, la référence 1 se rapporte au substrat structuré formé dans cet exemple, constitué d'une plaquette de silicium 3, d'une couche d'or 5, et d'une couche de résine photosensible 7. La référence 9 se rapporte à la liaison de la couche d'or avec un générateur de courant électrique pour l'électropolymérisation, la référence 10 à une microcuvette, et les références 11 et 13 se rapportent au copolymère de pyrrole (référence 11) et de N-éthylamine pyrrole (référence 13) formé par électrodéposition sur la couche d'or 5 au fond de la microcuvette 10.

Dans cet exemple, l'étape c) de fixation de la molécule biologique est une étape de fixation indirecte. Elle comprend la fixation d'un agent de réticulation sur la fonction $NH_2$ du N-éthylamine pyrrole électrodéposé sur le fond des microcuvettes.

L'agent de réticulation utilisé dans cet exemple est le succinimidyl 4-(p-maléimidophényl) butyrate) (SMPB) décrit précédemment.

Cette fixation est réalisée en formant une liaison covalence entre la fonction $NH_2$ du pyrrole fonctionnalisé et la fonction succinate du SMPB.

Elle est réalisée par trempage du substrat précédemment formé dans une solution $10^{-3}$M de SMPB dans un solvant (diméthylformamimide).

Le polypyrrole formé est insoluble dans cette solution et dans la majorité des solvants courants.

La figure 2 est un schéma d'une vue en coupe du substrat structuré ainsi obtenu. Sur ce schéma, la référence 1 se rapporte au substrat structuré représenté sur la figure 1, et la référence 15 se rapporte à l'agent de réticulation SMPB. Cette figure 2 montre aussi la réaction entre le groupe succinimide de l'agent de réticulation et la fonction amine du pyrrole.

On a donc réalisé dans cet exemple des microcuvettes recouvertes d'un polypyrrole présentant une fonctionnalisation de surface, grâce au SMBP, de groupements réactifs de type maléimide.

Ces groupements maléimide de SMBP permettent la fixation de la sonde biologique sur le film de polypyrrole précédemment électrodéposé.

La sonde biologique utilisée dans cet exemple est un mélange d'oligonucléotides fonctionnalisés avec un groupement thiol SH.

Les oligonucléotides ont été préparés par une synthèse automatisée classique, et fonctionnalisés avec un groupement thiol. Les oligonucléotides fonctionnalisés sont prélevés par micropipetage dans des micropuits et injectés dans les microcuvettes par l'intermédiaire d'un microrobot dispenseur.

La figure 3 est un schéma d'une vue en coupe du substrat structuré représenté sur la figure 2, illustrant la fixation de l'oligonucléotide sur l'agent de réticulation. Sur cette figure, la référence 1 se rapporte au substrat structuré formé dans cet exemple, les références 11 et 13, comme sur les figures 1 et 2, se rapportent au copolymère de pyrrole et de N-éthylamine pyrrole, la référence 15 à l'agent de réticulation SMBP représenté sur la figure 2 et la référence 17 se rapporte à un oligonucléotide. Cette figure 3 montre aussi la réaction entre la fonction maléimide de l'agent de réticulation et l'oligonucléotide -SH.

La densité de sonde a été analysée par fixation d'oligonucléotides marqués par une biotine (référence 19 sur la figure 3) et en utilisant une reconnaissance par la streptavidine Cy3 (référence 21 sur la figure 3).

L'analyse a été réalisée par une méthode classique de détection par fluorescence, appliquée au couple biotine-streptavidine.

<u>Exemple 2</u> : Fabrication d'une biopuce constituée notamment de sondes d'oligonucléotides greffées sur un polymère conducteur selon un second mode de réalisation du procédé de la présente invention.

Selon ce deuxième mode de réalisation, relatif notamment à l'étape a) du procédé de l'invention, une résine photosensible négative est déposée sur une plaquette de silicium recouverte d'un film naturel de $SiO_2$.

Comme dans l'exemple 1, des microcuvettes sont alors formées par photolithographie de telle manière que le fond des microcuvettes, appelées aussi ci-après sites, soit constitué par la couche d'oxyde de silicium.

On procède ensuite à une fonctionnalisation des sites par silanisation : cette fonctionnalisation est une étape collective du procédé de l'invention, elle est réalisée par trempage de la plaquette de silicium comportant les microcuvettes formées précédemment dans une solution d'un agent de silanisation fonctionnalisé avec un pyrrole dans un solvant approprié. L'agent de silanisation est le N-(3-(triméthoxysilyl)propyl) pyrrole, et le solvant est un mélange éthanol/eau (95/5) ou du toluène.

On obtient sur le fond des microcuvettes, ou sites, une monocouche de silane présentant un alignement régulier de sites pyrrole.

Cette monocouche est capable d'initier et de promouvoir l'adhésion d'un film de polypyrrole par électropolymérisation : elle forme une électrode de travail pour l'électropolymérisation collective du procédé de la présente invention.

L'électropolymérisation sur une telle monocouche est par exemple décrite dans l'article de R. Simon et Coll; J. Am. Chem. Soc. 1982, 104, 2031.

L'étape suivante est l'étape b) du procédé de l'invention, d'électropolymérisation d'un copolymère de pyrrole et de N-éthylamine pyrrole noté ci-après Py et Py-R-F, où R et F sont respectivement un groupement espaceur et une fonction chimique réactive.

La plaquette de silicium fonctionnalisée par le silane pyrrole constitue en fait l'anode d'une cellule d'électrolyse. Elle est plongée dans un bain électrolytique approprié, contenant les deux polymères, une contre-électrode et une électrode de référence.

Le bain électrolytique comprend outre Py et Py-R-F des sels électrolytiques de $Li^+$ dans un solvant eau/éthanol ou acétonitrile.

La contre-électrode est une électrode de platine. Au cours de l'électropolymérisation, les noyaux pyrrole et pyrrole substitué viennent s'insérer et se lier aux motifs pyrrole de la monocouche de silane.

La figure 4 en annexe illustre le produit ainsi obtenu, elle montre aussi la formation de liaisons covalentes entre les différents cycles pyrrole.

Sur cette figure, la référence 32 se rapporte à la plaquette de silicium, la référence 34 à la couche de résine photosensible, la référence 35 à une microcuvette, la référence 36 à la monocouche de silane, et la référence 38 à la couche de copolymère de pyrrole et de pyrrole fonctionnalisé.

La fabrication de la biopuce est achevée comme dans l'exemple 1 :

- réactions avec l'agent de réticulation bifonctionnel : étape collective,
- immobilisation des sondes d'oligonucléotides fonctionnalisés avec un groupement thiol (-S-H) par adressage mécanique avec un robot par impression à jets de liquide (tête piézoélectrique) de type GESIM ou avec un robot de type BROWN.

Exemple 3 : Fabrication d'une biopuce constituée notamment de sondes d'oligonucléotides greffés sur un polymère conducteur selon un troisième mode de réalisation du procédé de la présente invention.

Dans cet exemple de réalisation du procédé de la présente invention, les microcuvettes ont été réalisées par photolithographie d'une résine déposée sur une électrode d'or à la surface d'une plaquette de silice comme dans l'exemple 1 précédent.

Il a ensuite été procédé à une thiolisation de la couche d'or au fond des microcuvettes par un pyrrole fonctionnalisé avec un groupement -SH de formule suivante :

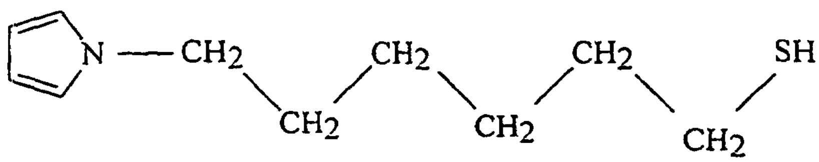

La réaction a été réalisée par trempage de la plaquette précitée dans une solution contenant le pyrrole fonctionnalisé avec un thiol dans un solvant comme le diméthylformamide (DMH) par exemple.

Le thiol s'est accroché sur l'or au fond des microcuvettes pour former une monocouche de pyrrole. L'ensemble couche d'or et pyrrole fixé sur celle-ci formant une électrode de travail pour l'électropolymérisation collective de l'étape b) du procédé de l'invention. En fait, l'échantillon sert d'anode pour l'amorçage collectif de l'électropolymérisation.

Les étapes b) et c) du procédé de l'invention ont alors été réalisées comme dans les exemples 1 et 2 précédents.

La figure 5 en annexe est un schéma illustrant le produit obtenu dans cet exemple. Il s'agit d'une vue en coupe d'un substrat structuré 40 comprenant une plaquette de silicium 42, une couche d'or 44, une couche de résine 46 photosensible dans laquelle sont formées des microcuvettes 48, une monocouche de pyrrole 50 accrochée sur l'or au fond des microcuvettes, et un film 52 de copolymère de pyrrole (Py) et de pyrrole fonctionnalisé

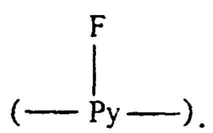

Sur cette figure, les flèches courbes indiquent l'électrodéposition du film précité sur le pyrrole 50 fonctionnalisé accroché par des groupements thiol sur l'or au fond des microcuvettes.

Exemple 4 : Compléments

Une autre approche consiste à utiliser un dépôt de polypyrrole fonctionnalisé, comme :

- soit un support d'immobilisation d'oligonucléotides,
- soit un support pour le démarrage d'une synthèse in situ de l'oligonucléotide.

Cette technique permet de remplacer avantageusement une étape de silanisation dans laquelle une monocouche est plus difficile à réaliser, par un film de polymère comportant une épaisseur et un nombre de sites fonctionnels bien contrôlés.

Pour ce faire, on réalise par électropolymérisation des films d'un copolymère comportant une proportion donnée de pyrrole fonctionnalisé par rapport au pyrrole. Ces films de polypyrrole, déposés sur une électrode d'or, au lieu de silicium ou verre, montrent une fluorescence parasite d'intensité bien inférieure à celle observée avec les autres substrats.

La fonctionnalisation peut se faire :

1. sur l'azote du pyrrole par une fonction $NH_2$ ou époxy, par exemple :

$Py-CH_2-CH_2-CH_2-NH_2$ ou 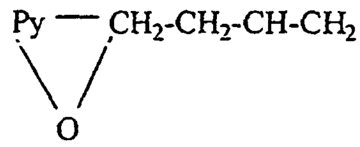

Ces fonctions peuvent servir à la fois à l'immobilisation de sondes et à la synthèse in situ ;

2. en position 1, 2 ou 3 du pyrrole par une fonction oxyamine ($R-ONH_2$) ou carbonyle (R, R'C=0 avec de préfrence $R'=CH_3$)). Dans ce cas, ces fonctions servent uniquement à l'immobiliation de sondes. L'oligonucléotide présente de préférence soit une fonction carbonyle, soit une fonction oxyamine selon le substrat. La réaction de couplage oxyamine-carbonyle présente l'avantage d'être très rapide et conduit à des temps d'immobilisation inférieurs à 10 minutes, contrairement à quelques heures comme dans le cas précédent ;

3. sur l'azote du pyrrole par un nucléotide comportant préférentiellement une base T. Cette fonctionnalisation sert à l'immobilisation de sondes comportant un groupement psoralène en 5'. Ce groupement réagit sous l'action de la lumière à 365 nm pour effectuer une cycloaddition entre la double liaison du psoralène et la double liaison 5, 6 de la Thymine ; le temps de réaction est relativement rapide : environ 15 min.

## Revendications

1. Procédé de fabrication d'une ébauche de biopuce, **caractérisé en ce qu'**il comprend les étapes suivantes successivement :

    a) structuration d'un substrat de manière à obtenir sur ce substrat des microcuvettes comprenant dans leur fond une couche d'un matériau capable d'initier et de promouvoir l'adhésion sur celle-ci d'un film d'un copolymère de pyrrole et de pyrrole fonctionnalisé par électropolymérisation,
    b) électropolymérisation collective, de manière à former un film électropolymérisé d'un copolymère de pyrrole et de pyrrole fonctionnalisé sur le fond desdites microcuvettes, sur la couche dudit matériau. à partir d'une solution de pyrrole et de pyrrole fonctionnalisé, en présence de réactifs chimiques appropriés pour ladite électropolymérisation.

2. Procédé de fabrication d'une biopuce comprenant la fabrication d'une ébauche de biopuce selon les étapes a) et b) de la revendication 1 et comprenant ensuite une étape c) de fixation directe, ou indirecte, d'une sonde biologique sur le pyrrole fonctionnalisé, par injection d'une solution de la sonde biologique, au choix dans une ou plusieurs microcuvette(s) en présence de réactifs chimiques nécessaires à la fixation directe, ou indirecte, de cette sonde biologique sur le pyrrole fonctionnalisé.

3. Procédé selon la revendication 1, dans lequel la couche du matériau capable d'initier et de promouvoir l'adhésion du film de polypyrrole par électropolymérisation étant une couche métallique, l'étape a) comprend une étape de dépôt de ladite couche métallique sur le substrat, et une étape de dépôt d'une couche de résine sur la couche métallique et de gravure de ladite couche de résine de manière à former les microcuvettes dont le fond est constitué au moins en partie de la couche métallique.

4. Procédé selon la revendication 3, dans lequel la couche métallique est une couche d'or.

5. Procédé selon la revendication 3 ou 4, dans lequel l'étape a) comprend en outre, une étape de traitement chimique de la couche d'or au fond des microcuvettes en présence d'un pyrrole fonctionnalisé avec un groupement thiol de manière à former une monocouche de pyrrole sur ladite couche d'or, au fond desdites microcuvettes.

6. Procédé selon la revendication 5, dans lequel le pyrrole fonctionnalisé avec un groupement thiol a la formule chimique suivante :

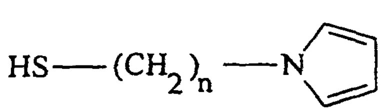

dans laquelle n a une valeur allant de 2 à 10.

7. Procédé selon l'une quelconque des revendications 1 à 6, dans lequel le substrat est une plaquette de silicium.

8. Procédé selon la revendication 1, dans lequel le substrat est une plaquette de silicium et dans lequel la couche capable d'initier et de promouvoir l'adhésion sur celle-ci du film de polypyrrole par électropolymérisation étant une couche de silane présentant un alignement de sites pyrrole, l'étape a) comprend une étape de dépôt d'une couche de résine sur la plaquette de silicium, ladite plaquette de silicium étant recouverte d'un film de $SiO_2$, et de gravure de ladite couche de résine de manière à former les microcuvettes dont le fond est constitué au moins en partie du film de $SiO_2$ ; et une étape de traitement des microcuvettes au moyen d'un agent de silanisation fonctionnalisé avec un pyrrole de manière à fixer sur le film de $SiO_2$, dans le fond des microcuvettes, la couche de silane présentant un alignement de sites pyrroles.

9. Procédé selon la revendication 8, dans lequel l'agent de silanisation est choisi dans un groupe comprenant le N-(3-(triméthoxy silyl)propyl) pyrrole, ou tout autre pyrrole fonctionnalisé avec un groupement $-SiCl_3$ ou $-Si(OMe)_3$.

10. Procédé selon l'une quelconque des revendications 1 à 9, dans lequel l'électropolymérisation collective est réalisée par trempage du substrat structuré obtenu à l'étape a) dans un bain électrolytique comprenant une solution de pyrrole, de pyrrole fonctionnalisé, et de réactifs chimiques appropriés pour l'électropolymérisation, en présence d'une contre-électrode qui trempe dans le bain électrolytique et est indépendante du substrat structuré, la couche de matériau capable d'initier et de promouvoir l'adhésion sur celle-ci du film de copolymère de pyrrole et de pyrrole fonctionnalisé formant une électrode de travail.

11. Procédé selon l'une quelconque des revendications 1 à 10, dans lequel le pyrrole fonctionnalisé est un pyrrole comportant un groupement choisi dans un ensemble comprenant un groupement $NH_2$, un groupement thiol, un groupement ester de la N-hydroxysuccinimide, un groupement triméthoxy silyl, un groupement carboxylique, aldéhyde, isothiocyanate.

12. Procédé selon l'une quelconque des revendications 1 à 10 dans lequel le pyrrole fonctionnalisé est choisi parmi un des composés suivants :

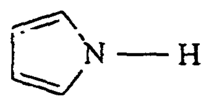

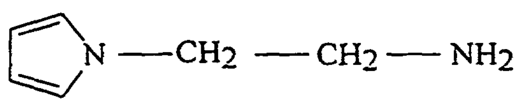

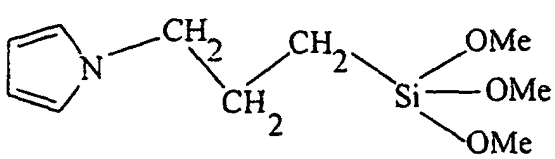

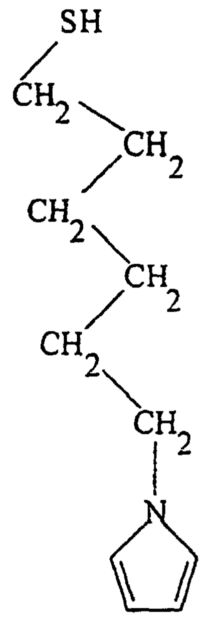

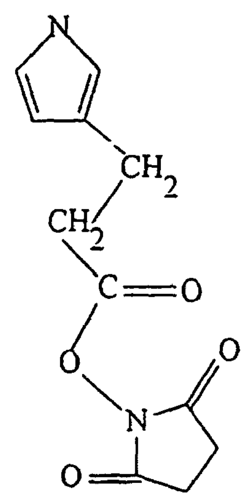

**13.** Procédé selon la revendication 2 dans lequel la fixation de la sonde biologique étant indirecte, ledit procédé comprend en outre, dans l'étape c), avant la fixation de la sonde biologique, une fixation collective d'un agent de réticulation sur le pyrrole fonctionnalisé, en présence de réactifs chimiques appropriés, ledit agent de réticulation comportant une première fonction permettant sa fixation sur le pyrrole fonctionnalisé, et une deuxième fonction permettant la fixation de la sonde biologique sur ledit agent de réticulation.

**14.** Procédé selon la revendication 13, dans lequel l'agent de réticulation est choisi dans un ensemble comprenant un dialdéhyde, un diisothiocyanate, un diacide, un anhydride succinique.

**15.** Procédé selon la revendication 13, dans lequel l'agent de réticulation est choisi parmi un des composés suivants :

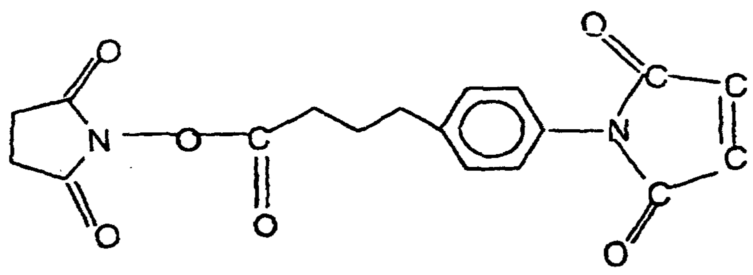

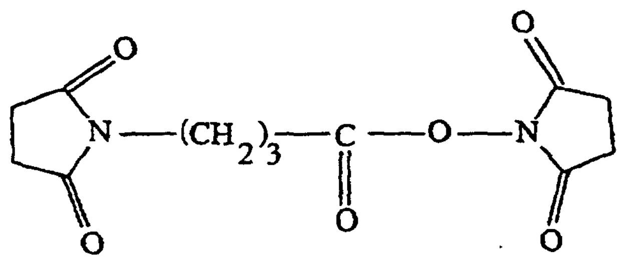

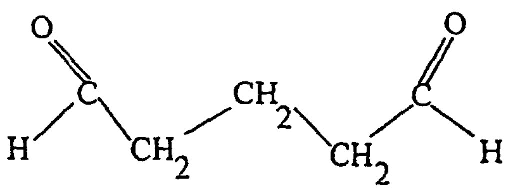

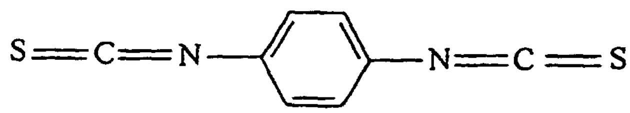

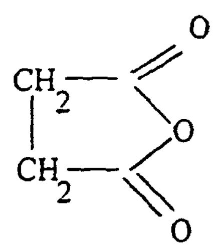

**16.** Procédé selon la revendication 2, dans lequel la sonde biologique est choisie parmi un oligonucléotide, un ADN, un ARN,un peptide, un glucide, un lipide, une protéine, un anticorps, un antigène.

**17.** Procédé selon la revendication 2, dans lequel la sonde biologique est un oligonucléotide fonctionnalisé pour être fixé soit directement, soit indirectement sur un pyrrole fonctionnalisé.

**18.** Procédé selon la revendication 17, dans lequel l'oligonucléotide est fonctionnalisé avec un groupement thiol.

**19.** Ebauche de biopuce comprenant dans cet ordre :

- un substrat (42),

- une couche (44) d'un matériau capable d'initier et de promouvoir l'adhésion sur celle-ci d'un film d'un copolymère de pyrrole et de pyrrole fonctionnalisé par électropolymérisation,
- une couche (46) de résine recouvrant ladite couche de matériau capable d'initier et de promouvoir l'adhésion sur celle-ci d'un film d'un copolymère du pyrrole et de pyrrole fonctionnalisé, dans laquelle des microcuvettes (48) ont été réalisées de telle sorte que le fond desdites microcuvettes est constitué au moins en partie de la couche (44) dudit matériau,
- une couche (52) d'un copolymère de pyrrole et de pyrrole fonctionnalisé, fixée sur ledit matériau constituant le fond desdites microcuvettes.

**20.** Biopuce comprenant dans cet ordre :

- une ébauche de biopuce selon la revendication 19, dans laquelle le substrat (42) est un substrat de silice, dans laquelle la couche de matériau capable d'initier et de promouvoir l'adhésion sur celle-ci d'un film d'un copolymère de pyrrole et de pyrrole fonctionnalisé est une couche (44) d'or ou une couche de silane présentant des sites pyrrole, et dans laquelle le pyrrole fonctionnalisé est lié ou non à un agent de réticulation bifonctionnel ; et
- un oligonucléotide fixé au fond des microcuvettes directement sur le pyrrole fonctionnalisé, ou indirectement sur le pyrrole fonctionnalisé par l'intermédiaire de l'agent de réticulation lié audit pyrrole.

## Claims

**1.** Method to produce a blank biochip, **characterized in that** it comprises the following successive steps:

a) structuring of a substrate so as to obtain on said substrate microtroughs comprising in their base a layer of a material capable of initiating and promoting the adhesion on said layer of a film of a pyrrole and a pyrrole copolymer functionalised by electropolymerisation,
b) collective electropolymerisation, so as to form an electropolymerised film of a pyrrole and functionalised pyrrole copolymer on the base of said microtroughs, on the layer of said material, using a pyrrole and functionalised pyrrole solution, in the presence of suitable chemical reagents for said electropolymerisation.

**2.** Method to produce a biochip comprising a blank biochip according to steps a) and b) of claim 1 and also comprising a step c) of direct or indirect fixation of a biological probe on the functionalised pyrrole, by injecting a biological probe solution, either in one or more microtroughs in the presence of chemical reagents required for the direct or indirect fixation of this biological probe on the functionalised pyrrole.

**3.** Method according to claim 1, wherein the layer of material capable of initiating and promoting the adhesion of the polypyrrole film by electropolymerisation being a metallic layer, step a) comprises a step of depositing said metallic layer on the substrate, and a step of depositing a layer of resin or polymer on the metallic layer and etching said resin layer so as to form microtroughs, the base being composed at least partly of the metallic layer.

**4.** Method according to claim 3, wherein the metallic layer is a gold layer.

**5.** Method according to claim 3 or 4, wherein step a) also comprises a step of chemically treating the gold layer at the base of the microtroughs in the presence of pyrrole functionalised with a thiol group so as to form a monolayer of pyrrole on said gold layer, at the base of said microtroughs.

**6.** Method according to claim 5, wherein the pyrrole functionalised with a thiol group has the following chemical formula:

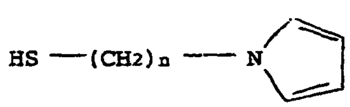

wherein n has a value ranging from 2 to 10.

**7.** Method according to any of claims 1 to 6, wherein the substrate is a silicon wafer.

**8.** Method according to claim 1, wherein the substrate is a silicon wafer and the layer capable of initiating and promoting the adhesion on said layer of the polypyrrole film by electropolymerisation being a layer of silane having an alignment of pyrrole sites, step a) comprises a step of depositing a layer of resin on the silicon wafer, which is coated with a $SiO_2$ film, and etching said resin layer so as to form the microtroughs in which the base is composed at least partly of the $SiO_2$ film; and a microtrough treatment step by means of a functionalised silanisation agent with a pyrrole so as to fix, on the $SiO_2$ film, in the base of the microtroughs, the silane layer having an alignment of pyrrole sites.

**9.** Method according to claim 8, wherein the silanisation agent is chosen in a group comprising N-(3-(trimethoxy silyl) propyl) pyrrole, or any other pyrrole functionalised with a $-SiCl_3$ or -Si(OMe)3 group.

**10.** Method according to any of claims 1 to 9, wherein the collective electropolymerisation is carried out by immersing the structured substrate obtained in step a) mentioned above in an electrolytic bath comprising a solution of pyrrole, functionalised pyrrole, and chemical reagents suitable for electropolymerisation, in the presence of a counterelectrode which is immersed in the electrolytic bath and is independent of the structured substrate, the layer of material capable of initiating and promoting the adhesion on said layer of the pyrrole and functionalised pyrrole copolymer film forming a working electrode.

**11.** Method according to any of claims 1 to 10, wherein the functionalised pyrrole is a pyrrole comprising a group chosen in a set comprising an $NH_2$ group, a thiol group, an N-hydroxysuccinimide ester group, a trimethoxy silyl group, a carboxyl, aldehyde and isothiocyanate group.

**12.** Method according to any of claims 1 to 10 wherein the functionalised pyrrole is chosen from one of the following compounds:

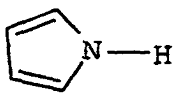

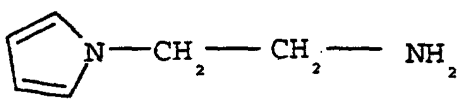

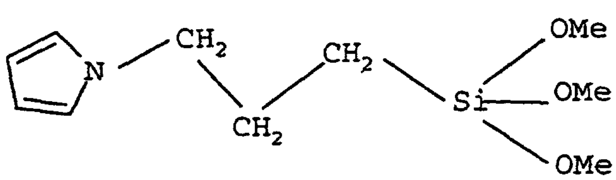

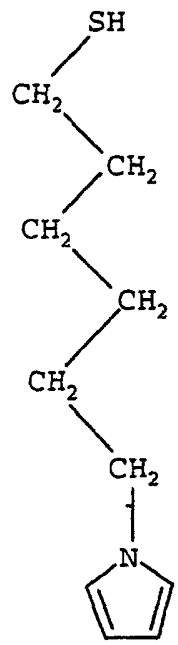

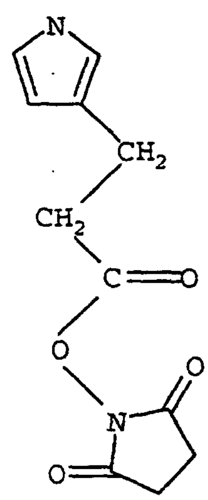

13. Method according to claim 2 wherein the fixation of the biological probe being indirect, said method also comprises, in step c); before the fixation of the biological probe, a collective fixation of a cross-linking agent on the functionalised pyrrole, in the presence of suitable chemical reagents, said cross-linking agent comprising a first function enabling its fixation onto the functionalised pyrrole, and a second function enabling the fixation of the biological probe on said cross-linking agent.

14. Method according to claim 13, wherein the cross-linking agent is chosen in a set comprising a dialdehyde, diisothiocyanate, a diacid, a succinic anhydride.

15. Method according to claim 13, wherein the cross-linking agent is chosen from one of the following compounds:

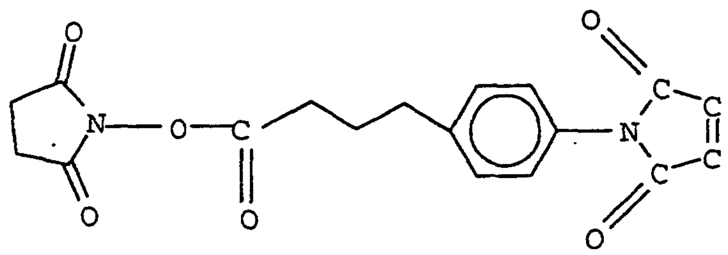

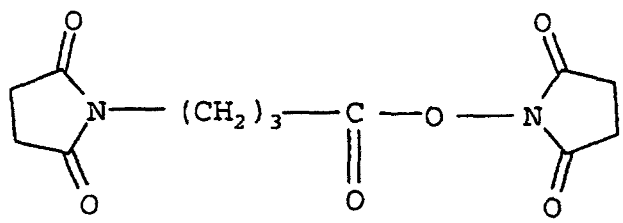

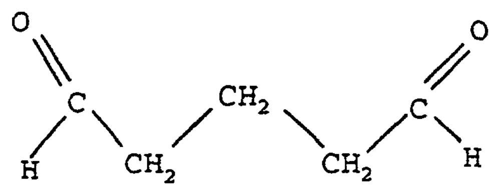

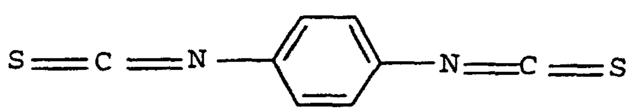

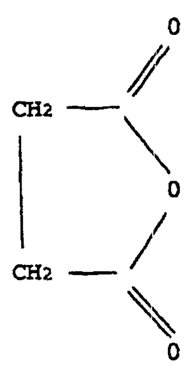

**16.** Method according to claim 2, wherein the biological probe is chosen from an oligonucleotide, a DNA, an RNA, a peptide, a glucide, a lipid, a protein, an antibody, an antigen.

**17.** Method according to claim 2, wherein the biological probe is an oligonucleotide functionalised for fixing either directly or indirectly on a functionalised pyrrole.

**18.** Method according to claim 17, wherein the oligonucleotide is functionalised with a thiol group.

**19.** Blank biochip comprising in this order:

- a substrate (42),
- a layer (44) of material capable of initiating and promoting on said layer the adhesion of a film of a pyrrole and pyrrole copolymer functionalised by electropolymerisation,
- a layer (46) of resin coating said layer of material capable of initiating and promoting the adhesion on said layer of a film of a pyrrole and functionalised pyrrole copolymer, in which microtroughs (48) have been produced so that the base of said microtroughs is composed at least partly of the layer (44) of said material,
- a layer (52) of a pyrrole and functionalised pyrrole copolymer, fixed on said material composing the base of said microtroughs.

**20.** Biochip comprising in this order:

- a biochip blank according to claim 19, in which the substrate (14) is a silica substrate, where the layer of material able to initiate and promote the adhesion thereon of a film of a pyrrole and a functionalised pyrrole copolymer is a layer (44) of gold or a silane layer having pyrrole sites and in which the functionalised pyrrole may or may not be linked with a biofunctional crosslinking agent and
- an oligonucleotide fixed to the base of the microtroughs in a direct manner to the functionalised pyrrole, or indirectly to the functionalised pyrrole by means of a crosslinking agent linked with said pyrrole.

## Patentansprüche

**1.** Verfahren zur Herstellung eines Biochip-Rohlings, **dadurch gekennzeichnet, dass** es die nachstehend angegebenen aufeinanderfolgenden Stufen umfasst:

a) Strukturierung eines Substrats, um auf diesem Substrat Mikroküvetten zu erzeugen, die in ihrem Boden eine Schicht aus einem Material umfassen, das die Haftung eines Films aus einem Copolymer von Pyrrol und funktionalisiertem Pyrrol daran durch Elektropolymerisation initiieren und fördern kann,
b) gemeinsame Elektropolymerisation zur Bildung eines elektropolymerisierten Films aus einem Copolymer von Pyrrol und funktionalisiertem Pyrrol auf dem Boden der genannten Mikroküvetten auf der Schicht aus dem genannten Material, ausgehend von einer Lösung von Pyrrol und funktionalisiertem Pyrrol in Gegenwart von chemischen Reagentien, die für die genannte Elektropolymerisation geeignet sind.

**2.** Verfahren zur Herstellung eines Biochips, das umfasst die Herstellung eines Biochip-Rohlings nach den Stufen (a) und (b) des Anspruchs 1 und anschließend eine Stufe (c) zur direkten oder indirekten Fixierung einer biologischen Sonde an dem funktionalisierten Pyrrol durch Injektion einer Lösung der biologischen Sonde, je nach Wunsch, in eine oder mehrere Mikroküvetten in Gegenwart von chemischen Reagentien, die für die direkte oder indirekte Fixierung dieser biologischen Sonde an dem funktionalisierten Pyrrol erforderlich sind.

**3.** Verfahren nach Anspruch 1, bei dem die Schicht des Materials, das die Haftung des Polystyrol-Films durch Elektropolymerisation initiieren und fördern kann, eine Metallschicht ist, dass die Stufe (a) eine Stufe zurAbscheidung der genannten Metallschicht auf dem Substrat und eine Stufe zur Abscheidung einer Harzschicht auf der Metallschicht und zur Ätzung der Harzschicht zur Bildung von Mikroküvetten umfasst, deren Boden mindestens zum Teil aus der Metallschicht besteht.

**4.** Verfahren nach Anspruch 3, bei dem die Metallschicht eine Goldschicht ist.

**5.** Verfahren nach Anspruch 3 oder 4, bei dem die Stufe (a) außerdem eine Stufe zur chemischen Behandlung der Goldschicht am Boden der Mikroküvetten in Gegenwart eines mit einer Thiolgruppe funktionalisierten Pyrrols umfasst zur Bildung einer Pyrrol-Monoschicht auf der genannten Goldschicht am Boden der genannten Mikroküvetten.

**6.** Verfahren nach Anspruch 5, bei dem das mit einer Thiolgruppe funktionalisierte Pyrrol die folgende chemische Formel hat:

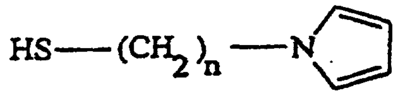

in der n einen Wert von 2 bis 10 hat.

**7.** Verfahren nach einem der Ansprüche 1 bis 6, bei dem das Substrat ein Silicium-Wafer ist.

**8.** Verfahren nach Anspruch 1, bei dem das Substrat ein Silicium-Wafer ist und bei dem die Schicht, welche die Haftung des Polypyrrol-Films an derselben durch Elektropolymerisation initiieren und fördern kann, eine Silanschicht ist, in der die Pyrrol-Zentren aufeinander ausgerichtet sind, die Stufe (a) eine Stufe zur Abscheidung einer Harzschicht auf dem Silicium-Wafer, wobei der genannte Silicium-Wafer von einem $SiO_2$-Flim bedeckt ist, und zur Ätzung der genannten Harzschicht zur Bildung der Mikroküvetten, deren Boden mindestens teilweise aus dem $SiO_2$-Film besteht, und eine Stufe zur Behandlung der Mikroküvetten mit einem Silanierungsmittel, das mit einem Pyrrol funktionalisiert ist, umfasst, um auf dem $SiO_2$-Film am Boden der Mikroküvetten die Sitanschicht zu fixieren, in der die Pyrrol-Zentren aufeinander ausgerichtet sind.

**9.** Verfahren nach Anspruch 8, bei dem das Silanierungsmittel ausgewählt wird aus der Gruppe, die N-(3-(Trimethoxysilyl)propyl)pyrrol oder irgendein anderes Pyrrol, das mit einer $-SiCl_3$- oder $-Si(OMe)_3$-Gruppe funktionalisiert ist, umfasst.

**10.** Verfahren nach einem der Ansprüche 1 bis 9, bei dem die gemeinsame Elektropolymerisation durchgeführt wird durch Eintauchen des in der Stufe (a) erhaltenen strukturierten Substrats in ein elektrolytisches Bad, das eine Lösung von Pyrrol, funktionalisiertem Pyrrol und chemischen Reagentien, die für die Elektropolymerisation geeignet sind, umfasst, in Gegenwart einer Gegenelektrode, die in das elektrolytische Bad eintaucht und unabhängig von dem strukturierten Substrat ist, wobei die Schicht aus dem Material, das die Haftung des Films aus dem Copolymer von Pyrrol und funktionalisiertem Pyrrol initiieren und fördern kann, eine Arbeitselektrode bildet

**11.** Verfahren nach einem der Ansprüche 1 bis 10, bei dem das funktionalisierte Pyrrol ein Pyrrol ist, das eine Gruppe umfasst, die ausgewählt wird aus einer Gesamtheit, die eine $NH_2$-Gruppe, eine Thiol-Gruppe, eine EsterGruppe von N-Hydroxysuccinimid, eine Trimeihoxysilyl-Gruppe, eine CarboxylGruppe, eine Aldehyd-Gruppe und eine Isothiocyanat-Gruppe umfasst.

**12.** Verfahren nach einem der Ansprüche 1 bis 10, bei dem das funktionalisierte Pyrrol ausgewählt wird aus einer der folgenden Verbindungen:

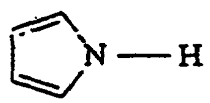

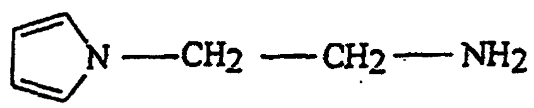

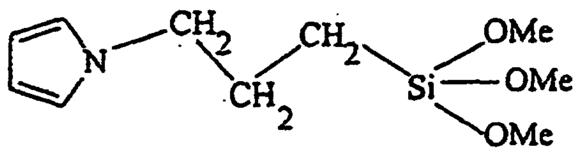

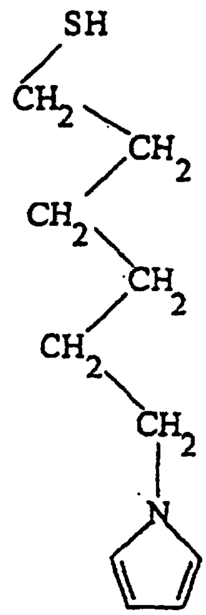

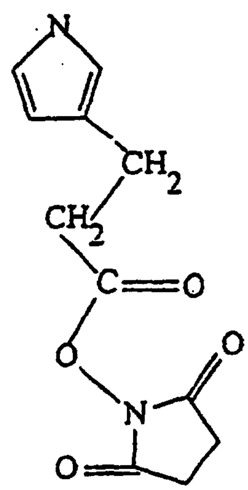

13. Verfahren nach Anspruch 2, bei dem die Fixierung der biologischen Sonde indirekt erfolgt, wobei das Verfahren in der Stufe (c) vor der Fixierung der biologischen Sonde außerdem eine kollektive Fixierung eines Vernetzungsmittels an dem funktionalisierten Pyrrol in Gegenwart von geeigneten chemischen Reagentien umfasst, wobei das genannte Vemetzungsmittel umfasst eine erste Punktion, die seine Fixierung an dem funktionalisierten Pyrrol erlaubt, und eine zweite Funktion, welche die Fixierung der biologischen Sonde an dem Vernetzungsmittel erlaubt.

14. Verfahren nach Anspruch 13, bei dem das Vernetzungsmittel ausgewählt wird aus einer Gesamtheit, die umfasst einen Dialdehyd, ein Diisothiocyanat, eine Disäure und ein Bemsteinsäureanhydrid.

15. Verfahren nach Anspruch 13, bei dem das Vemetzungsmittel ausgewählt wird aus einer der folgenden Verbindungen:

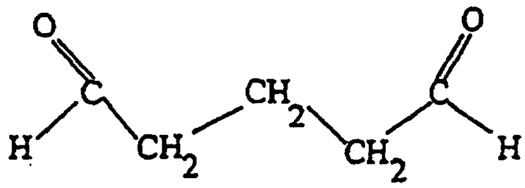

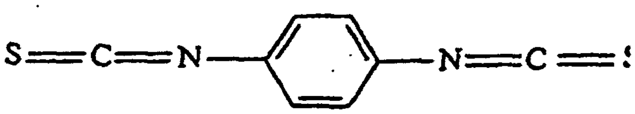

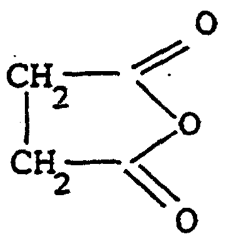

**16.** Verfahren nach Anspruch 2, bei dem die biologische Sonde ausgewählt wird aus einem Oligonucleotid, einer DNA, einer RNA, einem Peptid, einem Glucid, einem Lipid, einem Protein, einem Antikörper und einem Antigen.

**17.** Verfahren nach Anspruch 2, bei dem die biologische Sonde ein Oligonucleotid ist, das funktionalisiert ist, um direkt oder indirekt an einem funktionalisierten Pyrrol fixiert zu werden.

**18.** Verfahren nach Anspruch 17, bei dem das Oligonucleotid mit einer Thiol-Gruppe funktionalisiert ist.

**19.** Biochip-Rohling, der in der genannten Reihenfolge umfasst:

- ein Substrat (42),
- eine Schicht (44) aus einem Material, das die Haftung eines Films aus einem Copolymer von Pyrrol und funktionalisiertem Pyrrol an demselben durch Elektropolymerisation initiieren und fördern kann,
- eine Harzschicht (46), welche die Schicht aus dem Material bedeckt, das die Haftung eines Films aus einem Copolymer von Pyrrol und funktionalisiertem Pyrrol an demselben initiieren und fördern kann, in der Mikroküvetten (48) so gebildet worden sind, dass der Boden dieser Mikroküvetten mindestens zum Teil aus der Schicht (44) des genannten Materials besteht, und
- eine Schicht (52) aus einem Copolymer von Pyrrol und funktionalisiertem Pyrrol, die an dem Material fixiert ist, das den Boden der Mikroküvetten darstellt.

**20.** Biochip, der in der genannten Reihenfolge umfasst:

- einen Biochip-Rohling nach Anspruch 19, in dem das Substrat (42) ein Silicium-Substrat ist, in dem die Schicht aus dem Material, das die Haftung eines Films aus einem Copolymer von Pyrrol und funktionalisiertem Pyrrol an demselben initiieren und fördern kann, eine Goldschicht (44) oder eine Silanschicht ist, die Pyrrol-Zentren aufweist, und in der das funktionalisierte Pyrrol an ein bifunktionelles Vemetzungsmittel gebunden oder nicht gebunden ist; und
- ein Oligonucleotid, das am Boden der Mikroküvetten fixiert ist entweder direkt an dem funktionalisierten Pyrrol oder indirekt an dem funktionalisierten Pyrrol über ein Vernetzungsmittel, das an das Pyrrol gebunden ist.

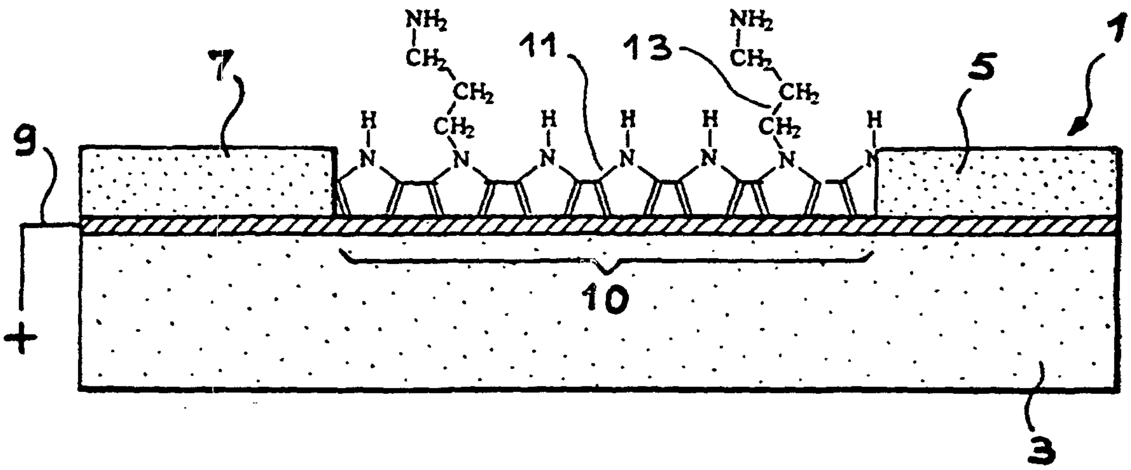


FIG. 1

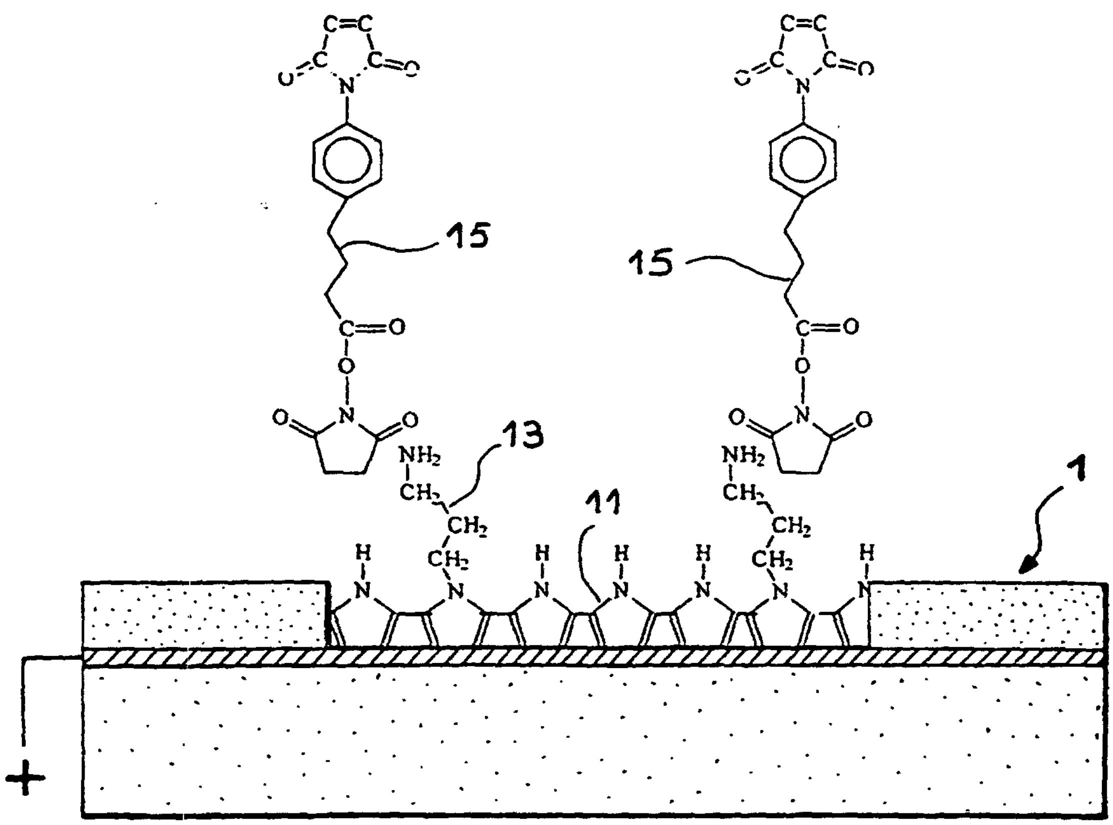


FIG. 2

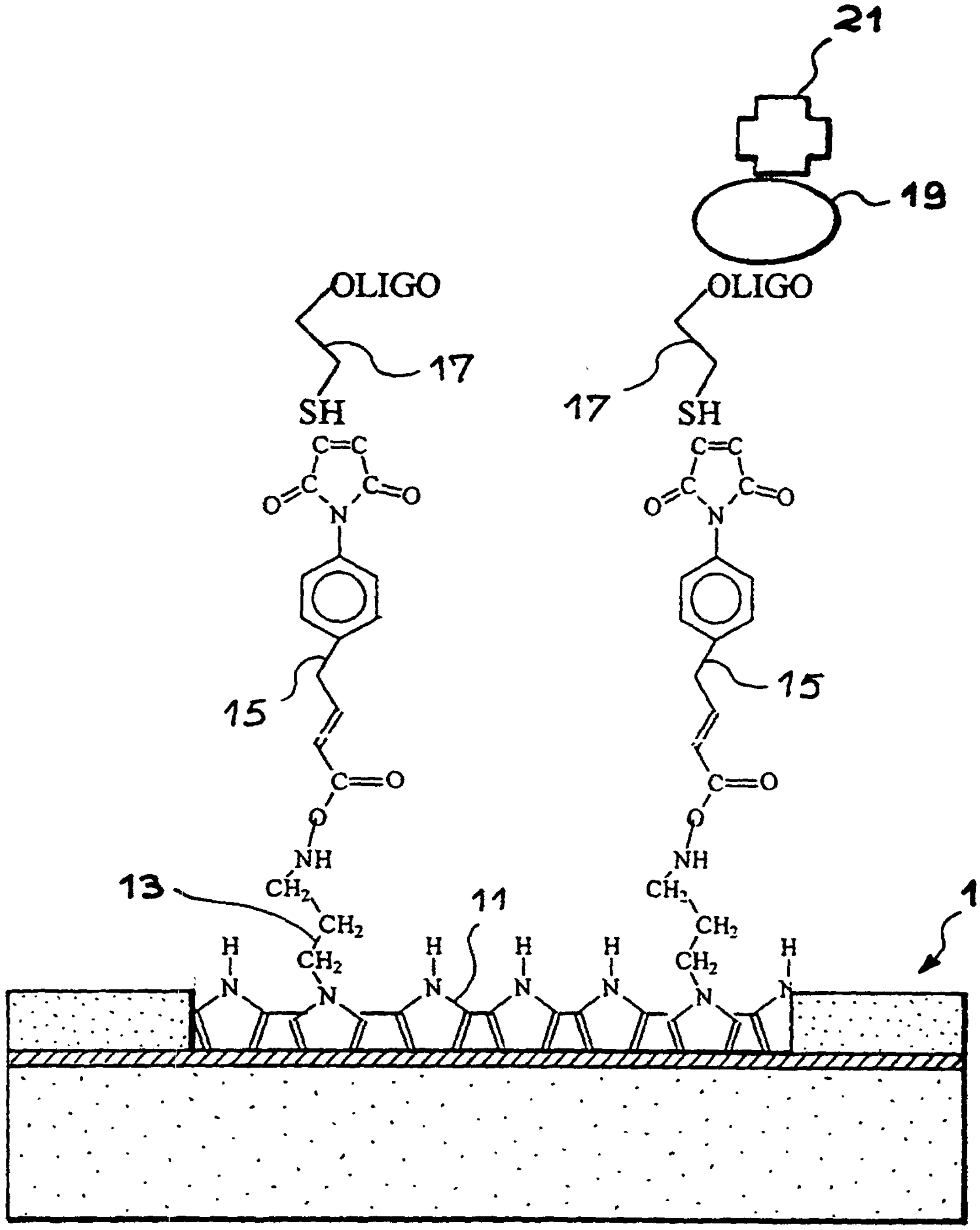


FIG. 3

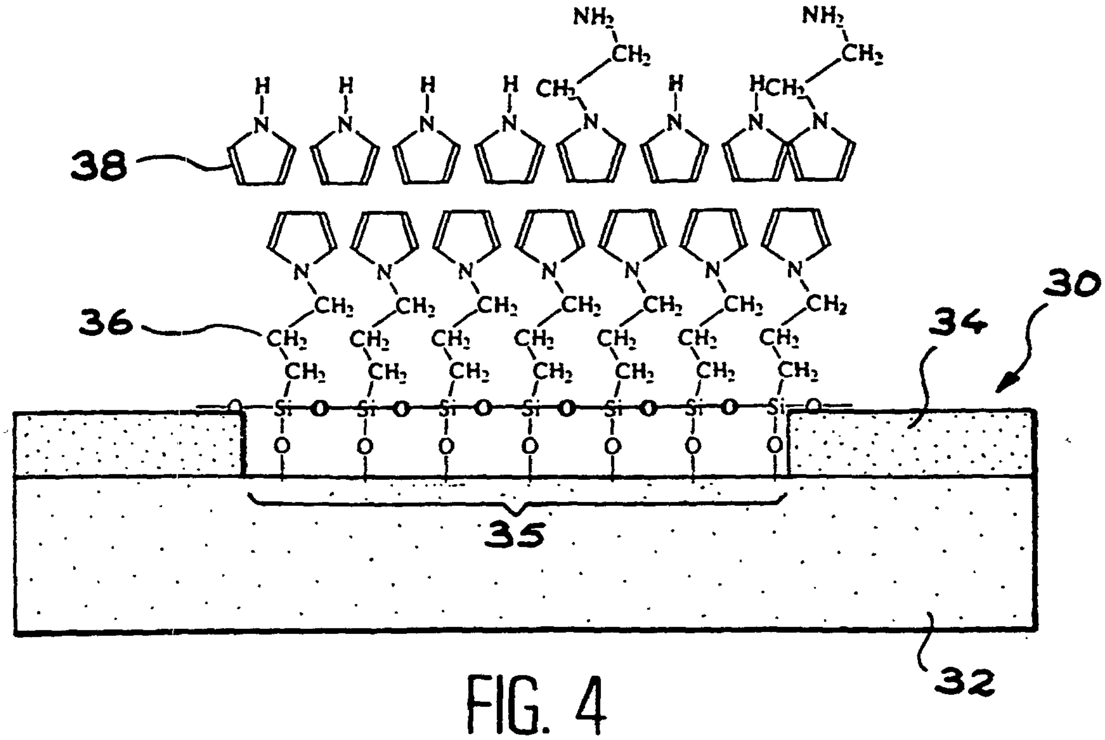
38
36
30
34
35
32

FIG. 4

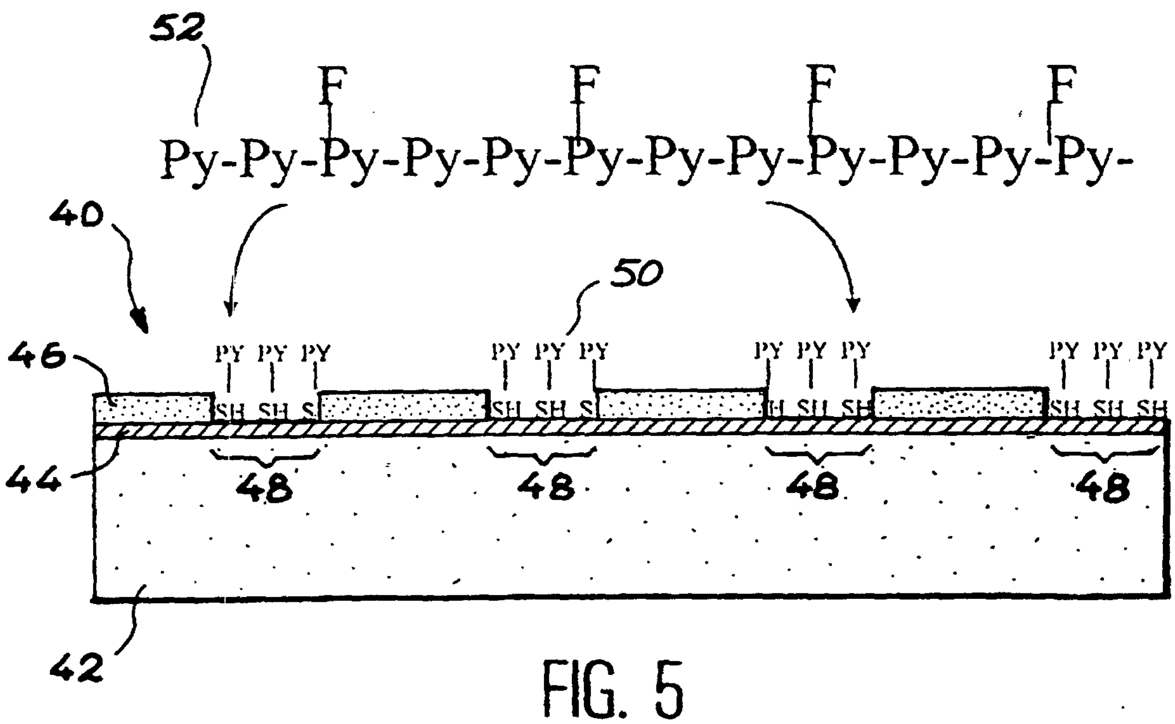
52
F F F F
Py-Py-Py-Py-Py-Py-Py-Py-Py-Py-Py-Py-
40
50
46
PY PY PY
SH SH S
44
48
42

FIG. 5